# EUROPEAN PATENT APPLICATION

(11) **EP 4 620 442 A1**
(43) Date of publication of application: **24.09.2025**
(21) Application number: 23891207.5
(22) Date of filing: 02.10.2023
(51) Int. Cl.: A61F 13/15

(54) **METHOD FOR MANUFACTURING INDIVIDUALLY-PACKAGED ABSORBENT ARTICLES, AND INDIVIDUALLY-PACKAGED ABSORBENT ARTICLE**

(30) Priority: 18.11.2022 JP 2022185096
(71) Applicant: DAIO PAPER CORPORATION, Shikokuchuo-shi Ehime 799-0492 (JP)
(72) Inventor: YOSHIOKA, Chisaki, Sakura-shi, Tochigi 329-1411 (JP)
(74) Representative: Epping - Hermann - Fischer
(86) International application number: PCT/JP2023/035951
(87) International publication number: WO 2024/106049

(57) **Abstract**

A method for producing an individually packaged absorbent article including a packaging sheet made of paper and an absorbent article individually packaged by the packaging sheet, includes disposing absorbent articles at intervals in a conveying direction on an inner surface of a packaging sheet long body, which becomes the packaging sheet made of paper, inwardly folding the packaging sheet long body along with the absorbent articles in an orthogonal direction orthogonal to the conveying direction, and compression bonding overlaid layers of the packaging sheet long body in a thickness direction by a pair of rolls that are heated, the compression bonding being applied at a position between the absorbent articles, wherein a temperature of at least one of the rolls is 110 to 180°C.

## Description

### TECHNICAL FIELD

The disclosures herein relate to methods for manufacturing individually packaged absorbent articles, and individually packaged absorbent articles.

### BACKGROUND ART

Generally, absorbent articles such as sanitary napkins, pantyliners, incontinence pads, and the like, are provided as individually packaged absorbent articles (individual packaging) in a state that they are individually packaged and sealed with packaging sheets for reasons such as hygiene in storage and convenience in carrying.

In manufacturing of such individually packaged absorbent articles, the absorbent articles are intermittently disposed on a long packaging sheet to convey. The long packaging sheet, along with the absorbent articles, is then inwardly folded in three in a direction perpendicular to a conveyance direction. Subsequently, hot pressing is applied to the long packaging sheet by two rolls positioned opposite each other at locations where there are no absorbent articles, forming a seal part (see, e.g., Patent Literature (PTL) 1).

### CITATION LIST

### PATENT LITERATURE

[PTL 1] Patent Publication No. 4275105

### SUMMARY OF THE INVENTION

### PROBLEMS TO BE SOLVED BY THE INVENTION

Resin films and nonwoven fabrics are mostly used as packaging sheets for individually packaged absorbent articles. These sheets are made of plastic materials. Therefore, when sealing both edge portions in a transverse direction of individually packaged absorbent articles, it is easy to ensure required seal strength because packaging sheet materials can be heat-sealed.

Conversely, in recent years, paper packaging sheets have been put to practical use from the viewpoint of reducing plastics. Since paper does not melt under normal conditions even when a temperature is raised, a bonding type in which the packaging sheets are physically engaged with each other or an adhesive or the like is used to form a seal on both edges of an individually packaged absorbent article including a paper packaging sheet. Such a sealing method requires separate preparation of a device different from an existing device, and therefore requires labor and cost for manufacturing.

In view of the above, an aspect of the present invention is a method for manufacturing an individually packaged absorbent article using a paper packaging sheet, wherein the seal parts on both edges of the individually packaged absorbent article are formed with appropriate strength without labor and cost.

### MEANS OF SOLVING THE PROBLEM

A method for producing an individually packaged absorbent article including a packaging sheet made of paper and an absorbent article individually packaged by the packaging sheet, includes disposing absorbent articles at intervals in a conveying direction on an inner surface of a packaging sheet long body, which becomes the packaging sheet made of paper, inwardly folding the packaging sheet long body along with the absorbent articles in an orthogonal direction orthogonal to the conveying direction, and compression bonding overlaid layers of the packaging sheet long body in a thickness direction by a pair of rolls that are heated, the compression bonding being applied at a position between the absorbent articles, wherein a temperature of at least one of the rolls is 110 to 180°C.

### EFFECTS OF THE INVENTION

According to one aspect of the present invention, in a method of manufacturing an individually packaged absorbent article using a paper packaging sheet, seal parts on both edge portions of the individually packaged absorbent article can be formed with sufficient strength without requiring labor and cost.

### BRIEF DESCRIPTION OF THE DRAWINGS

[FIG. 1] FIG. 1 is a plan view of an individually packaged absorbent article according to one embodiment of the present invention.
[FIG. 2] FIG. 2 is a plan view of the unfolded individually packaged absorbent article of FIG. 1.
[FIG. 3] FIG. 3 is a cross section of a line I-I of FIG. 1.
[FIG. 4] FIG. 4 is an enlarged view of a portion II of FIG. 1.
[FIG. 5] FIG. 5 is a drawing describing a joining step in a method of manufacturing the individually packaged absorbent article according to an embodiment of the present invention.
[FIG. 6] FIG. 6 is an enlarged view of the pressurizing part of a roll in FIG. 5.

### DESCRIPTION OF THE PREFERRED EMBODIMENTS

In the following, embodiments of the present invention will be described with reference to the accompanying drawings. In the drawings, the same or corresponding constituent elements are denoted with the same reference numerals, and description about the elements may be omitted unless particularly described.

### <BASIC STRUCTURE OF INDIVIDUALLY PACKAGED ABSORBENT ARTICLE>

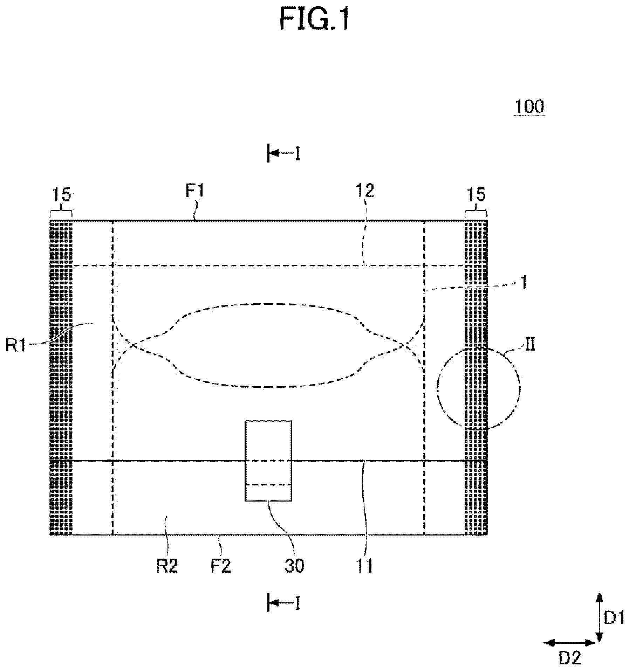

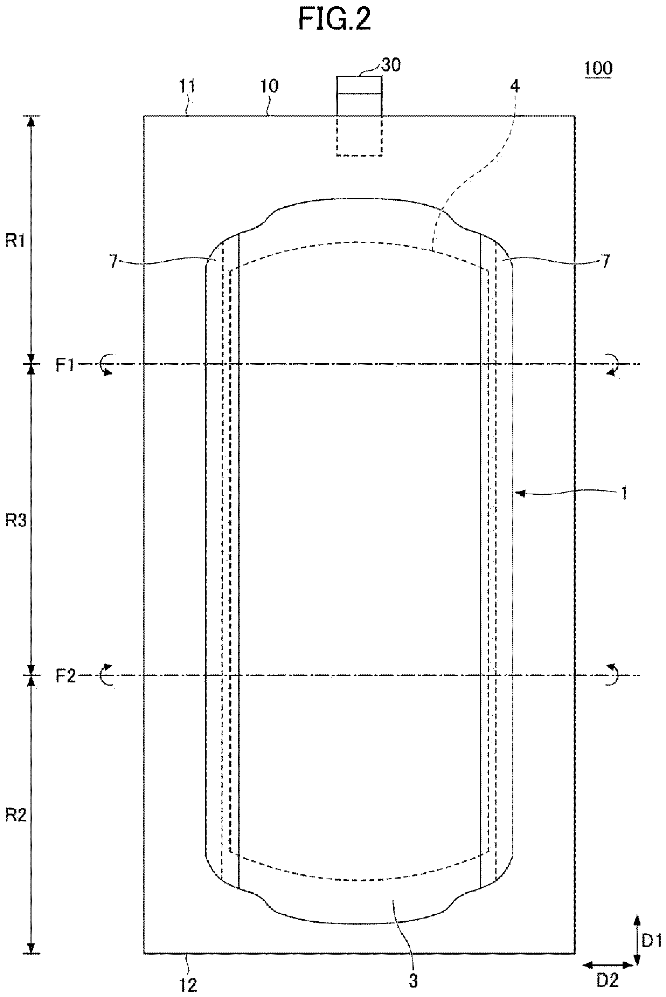

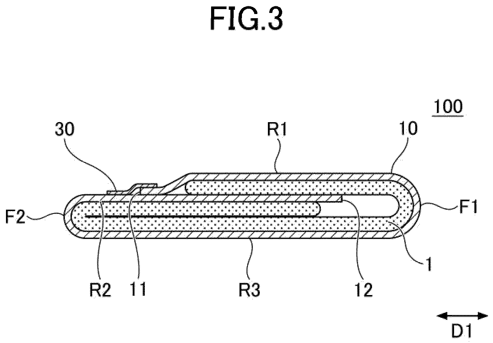

First, a basic structure of an individually packaged absorbent article will be described. FIG. 1 is a plan view of the individually packaged absorbent article 100. FIG. 2 is a plan view of the unfolded individually packaged absorbent article of FIG. 1, which is a drawing viewed from the inside of a packaging sheet 10 or from a surface of an absorbent article 1 facing a skin. FIG. 3 is a cross section of a line I-I of FIG. 1.

As shown in FIGS. 1 to 3, the individually packaged absorbent article 100 includes the packaging sheet 10 and the absorbent article 1 individually packaged by the packaging sheet 10. As shown in FIG. 2, the packaging sheet 10 may have an elongated shape when unfolded, and has a longitudinal direction (vertical direction) D1 and a transverse direction (horizontal direction) D2 perpendicular to the longitudinal direction. The longitudinal direction D1 and the transverse direction D2 of the packaging sheet 10 also correspond to the longitudinal direction and the transverse direction of the absorbent article 1, respectively.

### (ABSORBENT ARTICLE)

The absorbent article 1 packaged by the packaging sheet 10 may be a flat and elongated article to be attached so as to face an outlet of body fluid (menstrual blood, vaginal discharge, urine, etc.). Specific examples of the absorbent article 1 may be sanitary napkins, pantyliners, light incontinence pads, etc.

As shown in FIG. 2, for example, the absorbent article 1 may have a liquid-permeable top sheet 3, a liquid-impermeable back sheet (not shown), and an absorber 4 disposed between the top sheet 3 and the back sheet.

For the back sheet, at least a water-resistant sheet material such as an olefin resin sheet, including polyethylene or polypropylene, can be used. A laminated nonwoven fabric in which a nonwoven fabric is laminated on a polyethylene sheet or the like, or a laminated sheet of a nonwoven fabric in which a waterproof film is interposed so as to substantially ensure a liquid impermeability, can be used. In addition, a material having moisture permeability may also be used.

As the top sheet 3, a porous or non-porous nonwoven fabric or a porous plastic sheet or the like is suitably used. As the material fibers constituting the nonwoven fabric, for example, olefins such as polyethylene and polypropylene, synthetic fibers such as polyester and polyamide, regenerated fibers such as rayon and cupra, and mixed fibers of the aforementioned, and natural fibers such as cotton can be used alone or in combination of two or more types.

The absorber 4 is not limited as long as it is a material capable of absorbing and retaining body fluids, but preferably includes a cotton-like pulp and a water-absorbent polymer. Superabsorbent polymer (SAP), superabsorbent fiber (SAF), and a combination of SAP and SAF can be used as the water-absorbent polymer. Examples of pulp include cellulose fibers such as chemical pulp and dissolving pulp obtained from wood, as well as artificial cellulose fibers such as rayon and acetate. The pulp may be hardwood pulp obtained from hardwood, softwood pulp obtained from softwood, or a mixture of the hardwood pulp and the softwood pulp. Moreover, the pulp may be recycled pulp recycled from used pulp.

Thickness of the absorber 4 may be 0.5 to 25 mm. The absorber 4 may be designed with an inflated structure in a region corresponding to a body fluid outlet (body fluid outlet corresponding region) and a region facing a buttock groove behind the body fluid outlet corresponding region. The absorber 4 has a size and shape that do not protrude from the top sheet 3 and back sheet. At the front and rear end edge portions of the absorber, the outer edges of the back sheet and top sheet 3 are bonded using adhesives such as hot melt, or bonding methods such as heat sealing or ultrasonic sealing.

As shown in FIG. 2, on the lateral outside of the absorber 4, side sheets 7, 7 may be provided along the longitudinal direction D1 at both ends of the transverse direction D2. A water-repellent nonwoven fabric or a hydrophilic nonwoven fabric can be used as the side sheet 7.

In the example shown in FIGS. 1 to 3, the absorbent article 1 has no wings, or is what is called a wingless type, but it may be constructed as a winged article having wings extending in the both lateral directions. The wings may be formed by joining the side sheet and the back sheet.

Further, an anti-slip adhesive part to prevent the absorbent article 1 from slipping from underwear when attached may be provided on a surface facing the back sheet (a non-skin surface, or the surface facing the underwear when attached) of the absorbent article 1. The anti-slip adhesive part may be formed in a plurality of strips extending in the longitudinal direction D1 or the transverse direction D2.

A total length of the absorbent article 1 may be 140 to 430 mm, and a width of the absorbent article 1 (when having wings, the width of the body excluding the wings) may be 40 to 130 mm.

### (PACKAGING SHEET)

The packaging sheet 10 in the present embodiment is made of paper. Using a paper packaging sheet 10 can contribute to reduction of plastics and achievement of Sustainable Development Goals. In addition, paper can give a unique texture, for example, an appearance and touch of a gentle impression of a natural material. In the present description, paper refers to vegetable fibers or other fibers bonded with a binding agent to form a thin, flat sheet. In particular, paper made of vegetable fibers as a main raw material, for example, can refer to those containing 50% or more of vegetable fibers, especially cellulose fibers, preferably 80% or more. The vegetable fibers (pulp) used as the raw material of paper may include wood pulp, non-wood pulp, and waste paper pulp, and may be either mechanical pulp or chemical pulp. The pulp may be pulp recycled from components of absorbent articles and/or packaging sheets for absorbent articles. Further, additives may be added to the paper. Examples of paper include various types of paper such as Western paper, Japanese paper, converted paper, and synthetic paper. Furthermore, paper conventionally used for other purposes, such as newsprint paper, printing paper (including wood free paper), writing paper, drawing paper, packaging paper, thin paper, hybrid paper, and the like may be used. In the case of thin paper, thin Japanese vellum, Indian paper, rice paper, glassine paper, tissue paper, toilet paper, filter paper, and the like may be used.

In the present description, a term "paper packaging sheet" mainly refers to a sheet containing the above-mentioned paper. The paper packaging sheet may include not only a packaging sheet made of only paper, but also a laminated sheet in which paper and a sheet made of a material other than paper are laminated. When the packaging sheet 10 includes a sheet made of a material other than paper, the material other than paper may be a resin film, nonwoven fabric, or the like. However, when the packaging sheet 10 is made of only paper, it is particularly preferable from the viewpoint of reducing plastic and/or giving a natural texture unique to paper to a product.

The paper packaging sheet 10 may be subjected to some processing. This processing may include, for example, crepe finishing, embossing, calendering, water-repellent finishing, slit processing, ply processing, printing processing, and the like. Strength and/or flexibility of the paper can be improved by crepe finishing or embossing. The packaging sheet 10 used in the present embodiment may be crepe paper subjected to crepe finishing. Thus, even if the material is thin, the packaging sheet 10 is strong and flexible, and the surface of the packaging sheet has a moderate amount of fine protrusions and recesses, thereby improving the touch and ease of holding. In addition, when water-repellent finishing is performed on the back surface of the packaging sheet 10, a water-repellent containing a silicone resin, a paraffin resin, a fluororesin, or the like may be applied to at least one of an outer surface of the packaging sheet 10 (a surface where the absorbent article 1 is packaged and exposed to the outside) and an inner surface opposite to the outer surface. When the inner surface of the packaging sheet 10 is subjected to water-repellent processing, the adhesive portion for preventing slippage arranged on the back surface of the absorbent article 1 can be easily attached to and detached from the packaging sheet 10, so that a release sheet can be omitted. However, in the manufacturing method of forming the seal part by compression bonding using a heated roll according to the present embodiment, even if the packaging sheet 10 is subjected to water-repellent processing, the water-repellent may prevent forming a detachable seal part, and therefore, it is preferable that the seal part 15 (described later) on both edges of the individually packaged absorbent article 100 is not formed.

The size of the packaging sheet 10 depend on the size and shape of the absorbent article 1 to be packaged. For example, when the packaging sheet 10 is fully spread out (not folded back), a length of the longitudinal direction D1 (sometimes referred to simply as length) can be set to 100 to 450 mm, and a length of the transverse direction D2 (sometimes referred to simply as width) can be set to 70 to 250 mm. In the illustrated example, the packaging sheet 10 has a rectangular shape when it is unfolded, but may have a shape such as an oblong shape, for example.

### <PACKAGING STRUCTURE OF INDIVIDUALLY PACKAGED ABSORBENT ARTICLE>

As shown in FIGS. 1 to 3, the absorbent article 1 is folded and packaged with the packaging sheet 10 to form an individually packaged absorbent article 100. At the time of folding, as shown in FIG. 2, the absorbent article 1 is placed on the inner surface of the packaging sheet 10 so that the back sheet of the absorbent article 1 faces the inner surface of the packaging sheet 10. Both the packaging sheet 10 and the absorbent article 1 are folded in the longitudinal direction D1 at a first folding line F1 and a second folding line F2 along the width direction D2. More specifically, at the first folding line F1, a first region R1 including one end 11 of the longitudinal direction D1 of the packaging sheet 10 is folded back in the longitudinal direction D1, and at the second folding line F2, a second region R2 including the other end 12 of the longitudinal direction D1 of the packaging sheet 10 is folded back. The region between the first region R1 and the second region R2 of the packaging sheet 10 is a third region R3. In the illustrated example, the packaging sheet 10 is folded so that the second region R2 is folded first and the first region R1 overlaps the outer surface of the second region R2 (FIGS. 1 and 2), but the folding order of the first region R1 and the second region R2 may be reversed. After packaging the absorbent article 1 by inwardly tri-folding the packaging sheet 10 (folding the packaging sheet 10 in three) with the absorbent article 1, both edge portions of the transverse direction D2 are sealed along the longitudinal direction D1, and seal parts 15, 15 are formed.

Such a packaging form folded in three or more can be relatively easily formed, and the absorbent article 1 can be packaged hygienically, and the absorbent article 1 can be easily removed. The folding form is not limited to folding in three; it can also be folded in four or more, or in two.

Further, the individually packaged absorbent article 100 may be provided with a fastening tape 30 in the center of the transverse direction D2, near the end edge of the first region R1 (one end 11 of the packaging sheet 10). As shown in FIG. 1, the fastening tape 30 may be provided over the first region R1 and the second region R2 so as to straddle the one end 11 of the packaging sheet 10. By providing the fastening tape 30, the user can easily lift and pull the first region R1 to open the individually packaged absorbent article 100 by holding the fastening tape 30, making an opening process easier. At this time, the first region R1 is peeled off from the second region R2 located below it.

### (SEAL PART)

As shown in FIG. 1, in the individually packaged absorbent article 100, both edges of the transverse direction D2 where the absorbent article 1 does not exist are sealed, and seal parts 15, 15 are formed. In the present embodiment, the seal parts 15, 15 are formed by inserting the packaging sheet 10 into a pair of rolls and compression bonding overlaid layers of the packaging sheet 10 in a thickness direction by both rolls (further described later).

As shown in FIG. 1, the seal parts 15, 15 may be formed over the entire length of the longitudinal direction D1 of the individually packaged absorbent article 100. This is preferable from the viewpoint that it is possible to prevent dirt, dust, fingers or small objects from entering from the end edge in the transverse direction (sealing property is obtained).

A range of the transverse direction D2 provided with the seal part 15 may be within a range of 20 mm from the end edge of the transverse direction D2. The seal part 15 may be formed in the entire range, or in a part of the range, for example, at a position away from the end edge of the transverse direction D2. The length (width) of the transverse direction D2 of the seal part 15 is preferably 3 to 15 mm. An inner edge of the transverse direction D2 of the seal part 15 may extend linearly along the longitudinal direction D1, or in a curved shape, for example, in a wavy shape.

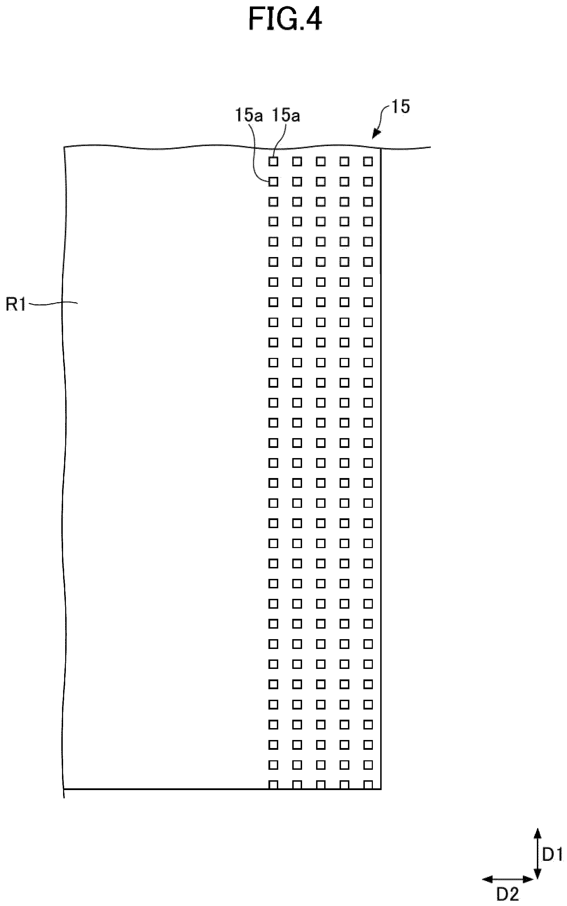

FIG. 4 is an enlarged view of a portion II including the seal part 15. As shown in FIG. 4, the seal part 15 may include a plurality of compression bonded parts (or joints) 15a, 15a, **...** which are spaced apart from each other in planar view. The respective compression bonded joint parts 15a, 15a, ... are portions where the different portions of the packaging sheet 10 are compression bonded in the thickness direction. In the illustrated example, portions other than the plurality of compression bonded parts 15a, 15a, **...** are non-joined parts where the different portions of the packaging sheet 10 are not joined to each other. Thus, because the compression bonded parts 15a, 15a, **...** are spaced apart from each other, it becomes easier to peel off the packaging sheets during opening, improving the opening ease. This is in contrast to when the different portions of the packaging sheet 10 are continuously bonded over the entire surface of the seal part 15. Additionally, this design prevents the edge portions of the individually packaged absorbent article 100 from becoming excessively hard and compromising the touch.

The plan view shape of each one of the compression bonded parts 15a, 15a, ... is a square in the example shown in FIG. 4, but it is not limited to the illustrated shape shown, and it may be, for example, a quadrilateral other than a square such as a rectangle, a parallelogram, a polygon other than a quadrilateral, a circle, an oval, a heart, a star, a drop, and the like. In the seal part 15, the size of one of the compression bonded parts 15a may be a square having sides of 0.2 to 2.5 mm or a size having an area equivalent to the aforementioned square.

### <METHOD FOR MANUFACTURING INDIVIDUALLY PACKAGED ABSORBENT ARTICLE>

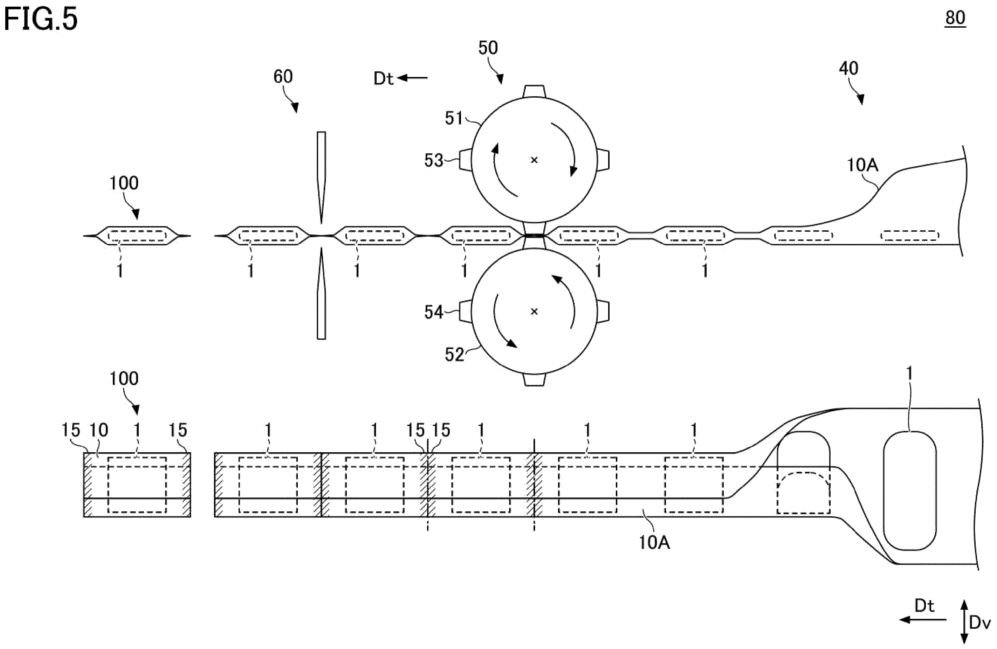

An embodiment of the present invention relates to a method for manufacturing the individually packaged absorbent article 100. A manufacturing apparatus 80 of an individually packaged absorbent article 100 is described referring to a schematic diagram (FIG. 5). First, a packaging sheet long body 10A to be a paper packaging sheet 10 (FIGS. 1 to 3) is transported in a conveyance direction Dt. The packaging sheet long body 10A can be obtained in a rolled-up state, and can be used by unrolling it from the roll. The packaging sheet long body 10A is transported along its longitudinal direction, and the absorbent articles 1, 1, ... are disposed on the inner surface of the packaging sheet long body 10A at intervals. The absorbent article 1 is arranged so that the longitudinal direction of the absorbent article 1 is along an orthogonal direction Dv orthogonal to the conveyance direction Dt. Subsequently, the packaging part 40 of the manufacturing apparatus 80 folds the packaging sheet long body 10A with the absorbent article 1 in the orthogonal direction Dv (i.e., the longitudinal direction D1 of the absorbent article 1) at a folding position along the conveyance direction Dt. It is preferable that the absorbent article 1 is inwardly folded in three so that the absorbent article 1 is not exposed after folding.

The absorbent articles 1, 1, ... disposed at intervals in the conveyance direction Dt are further conveyed to the seal forming part 50 of the apparatus 80 in a state of being packaged by the packaging sheet long body 10A. As shown in FIG. 5, the seal forming part 50 includes a pair of rolls 51, 52. In the seal forming part 50, at a position between the absorbent articles 1, 1, ... (a position where the absorbent article 1 is not arranged), the overlaid layers of the packaging sheet long body 10A are pressurized in a thickness direction by the pair of rolls 51, 52 to form a seal 15. The first roll 51 of the pair of rolls is provided with pressurizing parts 53, 53, ... along the circumferential direction, and similarly, the second roll 52 is provided with pressurizing parts 54, 54, ... along the circumferential direction. When the roll rotates, the pressurizing part 53 of the first roll 51 and the pressurizing part 54 of the second roll 52 face each other, and the packaging sheet long body 10A sandwiched between the rolls is pressurized in the thickness direction.

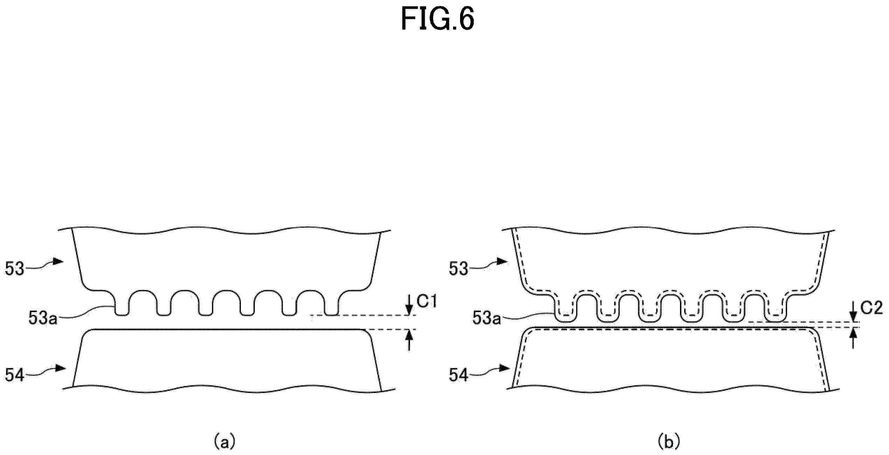

FIG. 6(a) shows an enlarged view of the pressurizing part 53 of the first roll 51 and the pressurizing part 54 of the second roll 52. In FIG. 6(a), an illustration of the packaging sheet long body 10A sandwiched between the rolls is omitted. As shown in FIGS. 6, compression bonded part forming protrusions 53a, 53a, ... are formed on the surface of the pressurizing part 53 of the first roll 51. Conversely, the second roll 52 is a roll (anvil roll) having a flat surface. Therefore, the size, shape, and arrangement of the compression bonded part forming protrusions 53a, 53a, ... of the first roll 51 can be designed in accordance with the desired size, shape, and arrangement of the compression bonded parts 15a, 15a, ... corresponding to the compression bonded parts 15a, 15a, ... (FIG. 4) of the seal part 15 of the individually packaged absorbent article 100 to be obtained. However, protrusions and recesses may be formed on the surfaces of both the first roll 51 and the second roll 52 so as to engage with each other.

In the present embodiment, at least one of the first rolls 51, 52 is heated. Therefore, at least one of the surfaces of the first rolls 51 and 52 may be heated. As an example of a heating means, an electric heating type may be used. For example, a resistance type heater (heat core) capable of generating heat may be embedded in the center of the rolls 51 and 52 or in the pressurizing parts 53, 53, ..., 54, 54, ... (FIGS. 5 and 6) of the rolls 51 and 52, respectively. In addition, an induction heating type, a heat medium circulation type, a steam heating type, or the like may be used. Among these methods, it is preferable to heat the rolls by the electric heating type because it is easy to process the surface of the rolls uniformly.

Thus, by using the pair of heated rolls 51 and 52 to form the seal part 15, the packaging sheet long body 10A can be joined in the thickness direction while heating and pressurizing. When the rolls are heated, the rolls slightly expand. FIGS. 6(a) and (b) are enlarged schematic views of the pressurizing parts 53 and 54 of the rolls 51 and 52 before and after expansion. After heating, as shown in FIG. 6(b), the pressurizing part 53 including the compression bonded part forming protrusions 53a, 53a, ... after expansion expands and approaches the pressurizing part 54, and the pressurizing part 54 also expands and approaches the pressurizing part 53. The dotted lines in FIG. 6(b) indicate the pressurizing parts 53 and 54 before heating. Thus, due to the expansion, a distance c2 between the pressurizing part 53 of the first roll 51 and the pressurizing part 54 of the second roll 52 in the heated state becomes narrower than a distance c1 between the pressurizing part 53 of the first roll 51 and the pressurizing part 54 of the second roll 52 before heating. That is, the distance between the first roll 51 and the second roll 52 after heating becomes smaller than that before heating. Therefore, by using heated rolls, the packaging sheet can be pressurized with a larger pressure than that by using unheated rolls. Since degree of expansion by heating is minute, the present embodiment is suitable for the case where a minute pressure adjustment is desired.

The compression bonding pressure by the pair of rolls may preferably be 0.1 to 10 MPa, more preferably 0.3 to 5 MPa. An increase in compression bonding pressure by heating can be 1 to 100 kPa.

Furthermore, by heating, fibers of the paper material contained in the packaging sheet long body 10A (or the packaging sheet 10) become likely to entangle each other. More specifically, functional groups (hydroxyl groups, etc.) exposed on the surface of cellulose fibers contained in the paper material become active, and chemical bonds, for example, hydrogen bonds, become likely to be formed between the cellulose fibers contained in the packaging sheet long body 10A (or the packaging sheet 10) which are stacked and opposed to each other. Therefore, the overlaid layers of the packaging sheet long body 10A can be firmly bonded even at a molecular level (microscopically). Therefore, it is not necessary to form a forced seal part to engage the overlaid layers of the packaging sheet long body 10A with each other.

Since the paper material does not contain a heat-sealing component, in the past, in manufacturing of individually packaged absorbent articles using paper packaging sheets, the seal part has been tried to be formed by physically engaging the packaging sheets or by using an adhesive. However, in the present embodiment, the seal part having an appropriate seal strength can be formed by using a heated roll. Therefore, it is not necessary to form a new surface pattern on the roll or to install an adhesive application device. Therefore, cost and labor can be saved. Moreover, when the seal part is formed by physically engaging the packaging sheets with each other, the material of the packaging sheets is burdened, and the packaging sheets may be compromised, and it is necessary to use a thicker material to prevent the compromise. According to the present embodiment, such concerns are also prevented.

The thickness of the paper packaging sheet long body 10A or the packaging sheet 10 may preferably be 40 to 200 µm, more preferably 70 to 150 µm, and further preferably 90 to 150 µm. The weight of the packaging sheet long body 10A or the packaging sheet 10 may preferably be 12 to 50 g/m², more preferably 15 to 40 g/m², and further preferably 15 to 35 g/m². According to the present embodiment, even when such a paper packaging sheet having a thin thickness and/or a small weight is used, the seal part 15 of the individually packaged absorbent article can be formed with an appropriate strength.

When forming the seal, it is preferable to raise the temperature of at least one roll, preferably both rolls. The temperature of the roll may be 110°C or more, preferably 120°C or more, more preferably 130°C or more, further preferably 135°C or more, and much further preferably 140°C or more. By setting the temperature to 90°C or more, a seal part of appropriate strength can be formed. The upper limit of the temperature may be such that the paper material is not denatured or compromised, for example, 180°C or less, and preferably 170°C or less. It is preferable that the roll is heated so that the temperature of at least the surfaces of the pressurizing parts 53 and 54 (in the case of the pressurizing part 53, the temperature of the surfaces of the compression bonded part forming protrusions 53a) becomes the above temperature. The above temperature can be a set temperature of the apparatus.

Furthermore, it is preferable that at least one, preferably both, of the rolls 51 and 52 are formed of a material having a linear thermal expansion coefficient of 5 to 50 µm/K. It is also preferable that at least the pressurizing part 53 of the first roll 51 and/or the pressurizing part 54 of the second roll 52 are formed of a material having a linear thermal expansion coefficient of 5 to 50 µm/K. The linear thermal expansion coefficient above may preferably be 5 to 25 µm/K. When the rolls 51 and 52 are composed of a material having such a linear thermal expansion coefficient, appropriate expansion of the rolls 51 and 52 can be obtained during heating. That is, the above-mentioned effect of narrowing the distance between the rolls 51 and 52 can be further improved, and excessive expansion can be prevented to facilitate adjustment of the compression bonding pressure during seal formation during operation.

The material constituting the roll is preferably an inorganic material including metal and ceramic, and metal is more preferable. The metal constituting the roll may be a single metal or an alloy. Specific examples include cemented carbide, stainless steel (SUS430, SUS304, etc.), carbon steel, high speed steel, die steel, iron, gold, copper, brass, aluminum, and the like. Of these, high speed steel and die steel are preferable because they have an appropriate linear thermal expansion coefficient and can appropriately expand the roll during heating.

### EXAMPLES

A paper packaging sheet (thickness: 90 µm, weight: 18 g/m²) having a length of 240 mm in the longitudinal direction was inwardly folded in three in the same manner as the packaging sheet 10 shown in FIGS. 1 and 2. The folded body was inserted between a pair of rolls made of high speed steel (linear thermal expansion coefficient: 11.2 µm/K) in the direction orthogonal to the folding direction and pressurized in the thickness direction to form a seal section having a width of 6 mm. As a pair of rolls, as described with reference to Fig. 6, one of the rolls has compression bonded part forming protrusion on the surface, and the other is a flat roll with no protrusions and recesses on the surface. The set roll pressure without heating was 3.3 MPa, and the rotation speed of the roll was 350 revolutions per minute.

Then, the seal part was formed by changing the temperature of the surface of the roll (the temperature of the pressurizing part), and the sealing property of the seal part at each temperature was evaluated visually. The evaluation was performed according to the following criteria.
∘: There were no openings.
△: There was an opening less than 5 mm.
×: There was an opening more than 5 mm.

Opening refers to a condition in which the end edge of the packaging sheet, which is placed outside and exposed when inwardly folded in three, is not joined to the packaging sheet stacked under it. Of the above, "o" and "△" are an allowable range for the seal part.

**[Table 1]**

| Roll Temperature | 100°C | 120°C | 130°C | 140°C | 150°C |
|---|---|---|---|---|---|
| Sealing Property | × | Δ | Δ | ○ | ○ |

As shown in Table 1, in the example of the metal roll (high-speed steel roll) in the present embodiment, it was found that the sealing property of the seal part can be improved by setting the surface temperature of the roll to 130°C or more, more preferably 140°C or more.

Although the present invention has been described based on the embodiments described above, the present invention is not limited to these embodiments, and various variations and modifications may be made without departing from the scope of the present invention. Moreover, the above-described components can be combined optionally.

Further, specific aspects of the present invention will be described below.

### (Clause 1)

In an aspect according to Clause 1, a method for producing an individually packaged absorbent article including a packaging sheet made of paper and an absorbent article individually packaged by the packaging sheet, includes disposing absorbent articles at intervals in a conveying direction on an inner surface of a packaging sheet long body, which becomes the packaging sheet made of paper, inwardly folding the packaging sheet long body along with the absorbent articles in an orthogonal direction orthogonal to the conveying direction, and compression bonding overlaid layers of the packaging sheet long body in a thickness direction by a pair of rolls that are heated, the compression bonding being applied at a position between the absorbent articles, wherein a temperature of at least one of the rolls is 110 to 180°C.

In the manufacturing of the individually packaged absorbent article, when a packaging sheet made of paper is used, it is not possible to heat-seal to form a seal like a packaging sheet made of a plastic material. Therefore, the sealing methods used in the past have been limited to physical engagement between the packaging sheets or use of an adhesive. Conversely, the present inventors have found, as a result of diligent research, that as the aspect of Clause 1 by compression bonding the overlaid layers of the packaging sheet long body (or the packaging sheet) to each other using a pair of rolls heated at a predetermined temperature, even if the packaging sheet is made of paper, it is possible to form a seal of appropriate strength, that is, it is possible to form a seal having both an ability to prevent unintended opening before use (sealing property) and an ability to easily unfold the bonded layers of the packaging sheet when they are opened (opening ease).

As the rolls are heated, the rolls slightly expand, and the distance between the pair of rolls can be narrower, so that a pressure during compression bonding can be increased. As the pressure increases, the cellulose fibers contained in each of opposing layers of the paper packaging sheet become likely to entangle each other, and the seal strength between the overlaid layers of the packaging sheets can be increased. In addition, since the temperature of the packaging sheet is also raised by heating the rolls, activity of functional groups on the surface of the cellulose fibers is increased, and chemical bonds (hydrogen bonds, etc.) become likely to be formed between the cellulose fibers contained in each of the opposing packaging sheets. Therefore, the packaging sheets can be bonded more firmly at the molecular level, and the strength of the seal at both edges can be increased.

### (Clause 2)

In an aspect according to Clause 2, a linear thermal expansion coefficient of at least one of the rolls is 5 to 50 µm/K.

According to the aspect of Clause 2, the roll expands, and the above-mentioned effect of narrowing the distance between the pair of rolls can be further improved.

### (Clause 3)

In an aspect according to Clause 3, the compression bonding is performed without using an adhesive.

As described above, a seal having sufficient strength can be formed even in the case of a paper packaging sheet, and therefore, according to the aspect of Clause 3, it is possible to save a cost of an adhesive and the labor of applying the adhesive, which are conventionally often used in the case of the paper packaging sheet.

### (Clause 4)

In an aspect according to Clause 4, a plurality of compression bonded part forming protrusions are formed on a surface of one roll, and a surface of the other roll is flat.

According to the aspect of Clause 4, in which the compression bonded part forming protrusions are formed on only one of the rolls, it is less likely to form mechanical engagement between the overlaid layers of the packaging sheet, compared with the sealing means in which protrusions and recesses are formed on both surfaces of the pair of rolls and the protrusions and recesses are formed to engage with each other. However, in the present embodiment, by heating the rolls, a seal of sufficient strength can be formed even if the rolls are not of an engaging type.

### (Clause 5)

In an aspect according to Clause 5, the packaging sheet made of paper has a thickness of 40 to 200 µm.

According to the aspect of Clause 5, even a relatively thin packaging sheet can be more reliably sealed.

### (Clause 6)

In an aspect according to Clause 6, an individually packaged absorbent article includes a packaging sheet made of paper and an absorbent article individually packaged by the packaging sheet, wherein while the absorbent article is disposed on an inner surface of the packaging sheet, the packaging sheet is inwardly folded along with the absorbent article in a longitudinal direction of the packaging sheet, and both edge portions in a transverse direction orthogonal to the longitudinal direction form a seal; and the seal is formed by thermocompression bonding at 110 to 180°C.

According to the aspect of Clause 6, even a paper packaging sheet can be provided with a seal part having an appropriate seal strength, and an individualized absorbent article with reduced cost and labor can be provided.

This application claims priority based on Japanese Patent Application no. 2022-185096, filed on November 18, 2022, the entire contents of which are incorporated herein.

### REFERENCE SIGNS LIST

1 absorbent article
3 top sheet
4 absorber
7 side sheet
10 packaging sheet
11 one end in the longitudinal direction
12 the other end in the longitudinal direction
15 seal part
15a compression bonded part
30 fastening tape
40 packaging part
50 seal forming part
60 cutting part
80 individually packaged absorbent article manufacturing apparatus
100 individually packaged absorbent article
D1 longitudinal direction of packaging sheet (opening direction)
D2 transverse direction of packaging sheet (width direction)
F1 first folding line
F2 second folding line
R1 first region
R2 second region
R3 third region

## Claims

1. A method for producing an individually packaged absorbent article including a packaging sheet made of paper and an absorbent article individually packaged by the packaging sheet, comprising:
disposing absorbent articles at intervals in a conveying direction on an inner surface of a packaging sheet long body, which becomes the packaging sheet made of paper;
inwardly folding the packaging sheet long body along with the absorbent articles in an orthogonal direction orthogonal to the conveying direction; and
compression bonding overlaid layers of the packaging sheet long body in a thickness direction by a pair of rolls that are heated, the compression bonding being applied at a position between the absorbent articles,
wherein a temperature of at least one of the rolls is 110 to 180°C.

2. The method according to claim 1, wherein a linear thermal expansion coefficient of at least one of the rolls is 5 to 50 µm/K.

3. The method according to claim 1 or 2, wherein the compression bonding is performed without using an adhesive.

4. The method according to claim 1 or 2, wherein a plurality of compression bonded part forming protrusions are formed on a surface of one roll, and a surface of the other roll is flat.

5. The method according to claim 1 or 2, wherein the packaging sheet made of paper has a thickness of 40 to 200 µm.

6. An individually packaged absorbent article, including a packaging sheet made of paper and an absorbent article individually packaged by the packaging sheet, wherein:
while the absorbent article is disposed on an inner surface of the packaging sheet, the packaging sheet is inwardly folded along with the absorbent article in a longitudinal direction of the packaging sheet, and both edge portions in a transverse direction orthogonal to the longitudinal direction form a seal; and
the seal is formed by thermocompression bonding at 110 to 180°C.
